# EUROPÄISCHE PATENTSCHRIFT

(11) **EP 1 808 189 B1**
(45) Veröffentlichungstag und Bekanntmachung des Hinweises auf die Patenterteilung: **17.08.2016**
(21) Anmeldenummer: 07000065.8
(22) Anmeldetag: 03.01.2007
(51) Int. Cl.: A61B 42/00, A61L 31/14, A61L 31/10

(54) **Prophylaxeartikel**
Prophylaxis items
Article de prophylaxie

(30) Priorität: 11.01.2006 AT 372006
(43) Veröffentlichungstag der Anmeldung: 18.07.2007
(73) Patentinhaber: Semperit Aktiengesellschaft Holding, 1031 Wien (AT)
(72) Erfinder: Schaller, Raimund, Dipl.-Ing. Dr., 2620 Neunkirchen (AT); Jirovsky, Gerhard, Dkfm., 2326 Maria Lanzendorf (AT)
(74) Vertreter: Ofner, Clemens

(56) Entgegenhaltungen:
- EP-A- 0 306 389
- EP-A- 1 479 355
- WO-A-00/25840
- DE-U1- 20 100 269

## Beschreibung

Die Erfindung betrifft einen mehrschichtigen Prophylaxeartikel in Form eines Handschuhs, insbesondere eines medizinischen Handschuhs, oder Kondoms aus zumindest einer elastomeren Träger- und zumindest bereichsweise einer Gleitschicht mit jeweils einer inneren und einer äußeren Oberfläche, wobei die äußere Oberfläche der Gleitschicht der inneren Oberfläche der Trägerschicht zugewandt ist und in der Gleitschicht Mikrokapseln enthalten sind, die zumindest einen Wirkstoff und/oder Farbstoff enthalten, sowie eine Zusammensetzung für die Bildung einer Gleitschicht eines Prophylaxeartikels in Form eines Handschuhs oder Kondoms und deren Verwendung.

Medizinische Handschuhe, sei es in Form von Untersuchungs- oder in Form von Operationshandschuhen, sind mittlerweile standardmäßige Ausrüstungen in der medizinischen Versorgung. Bei der Verwendung von medizinischen Handschuhen treten unterschiedliche Problemstellungen auf. Einerseits zeigen einige Verwender von medizinischen Handschuhen allergische Reaktionen auf Bestandteile der Innenschicht, insbesondere der Gleitschicht des Handschuhs. Andererseits erweist es sich als problematisch, wenn die Gleitschicht zu geringe Gleiteigenschaften aufweist und somit für den Verwender ein Anziehen des Handschuhs, vor allem mit feuchten Händen, beinahe unmöglich wird. Und zudem besteht die Gefahr, dass bei einer Beschädigung der Oberfläche eines medizinischen Handschuhs potentiell gefährliche Mikroorganismen durch die Schutzschicht des Prophylaxeartikels auf die Haut des Verwenders des Handschuhs treten können.

Aus dem Stand der Technik, wie z.B. der US 5,395,666 sind Eigenschaften eines Handschuhs bekannt, welche die Anziehbarkeit desselben verbessern sollen. In den erfindungsgemäßen Handschuhen werden Mikropartikel in der Größe von 4 bis 20 µm an der Innenseite des Handschuhs aufgetragen, wobei deren Durchmesser nicht mehr als 5 % der Dicke des flexiblen Elastomerartikels beträgt. Die Mikropartikel sind Kieselgelpartikel, welche vorzugsweise eine Größe von 5 bis 12 µm aufweisen. Sie weisen eine regelmäßige Form ohne Ecken und Kanten auf und zeigen bevorzugt amphotere oder kationische Oberflächeneigenschaften.

Eine Beschichtung mit Mikrokapseln für Handschuhe ist aus der US 2005/0066414 A1 bekannt, wobei die Zusammensetzung für die Beschichtung Mikrokapseln, Wasser und Polyurethan umfasst und die Mikrokapseln Kohlenwasserstoffe niedriger Viskosität, Duftstoffe, Vitamine und eine Mikrokapselbeschichtung beinhalten. Die Mikrokapselbeschichtung besteht aus Polyacetatharnstoff. In einer alternativen Ausführungsform umfassen die Mikrokapseln Polyisobuten, Vanilleduft, Vitamin A, Palmitate, Vitamin E-Acetat und eine Mikrokapselbeschichtung, wobei diese aus Polyoxymethylenharnstoff gebildet ist. Es werden Mikrokapseln im Umfang von 1 bis 5 Gew.-% der Tauchlösung, bestehend aus Wasser und Polyurethan, beigemischt.

Die EP 1 479 355 A2 beschreibt einen mehrschichtigen medizinischen Handschuh aus einer elastomeren Trägerschicht, mit einer inneren und einer äußeren Oberfläche, wobei auf der inneren Oberfläche zumindest in einem Teilbereich eine Gleitschicht aus einem polymeren Material mit einer inneren und einer, der inneren Oberfläche der Trägerschicht zugewandten, äußeren Oberfläche angeordnet ist. Auf der inneren Oberfläche der Trägerschicht und/oder zwischen der Trägerschicht und der Gleitschicht und/oder in der Gleitschicht und/oder auf der inneren Oberfläche der Gleitschicht ist zumindest im Teilbereich zumindest ein Wirkstoff und/oder Farbstoff innerhalb von mit einem maximalen Durchmesser zwischen 10 µm und 500 µm angeordnet und/oder ist in dem zumindest einen Teilbereich zwischen der Trägerschicht und der Gleitschicht eine Schicht, umfassend den zumindest einen Wirkstoff und/oder Farbstoff, angeordnet ist, wobei die Gleitschicht regelmäßig wiederkehrende Erhöhungen bzw. Vertiefungen mit unregelmäßiger Form, hergestellt durch raschen Flüssigkeitsentzug aus der Gleitschicht, aufweist, wobei sich ein Anteil der Vertiefungen von 20 % bis 95 %, bezogen auf die Gesamtzahl der Vertiefungen, über die gesamte Dicke der Gleitschicht erstreckt.

Die EP 0 306 389 A1 beschreibt einen Handschuh bzw. ein Kondom umfassend mindestens zwei aufeinander angebrachte Schichten aus elastomerem Material, wobei zwischen diesen Schichten zumindest eine aktive Substanz in gegen Keime oder Spermien in Mikrokapseln eingeschlossen angeordnet ist. Die Mikrokapseln weisen eine Größenordnung von 5 µm und 50 µm auf.

Aufgabe der Erfindung ist es einen Prophylaxeartikel mit verbesserten Eigenschaften zur Verfügung zu stellen. Weiters ist es Teilaufgabe der Erfindung die Haftbarkeit der Mikrokapseln an der Innenseite des Prophylaxeartikels zu verbessern.

Die Aufgabe der Erfindung wird jeweils eigenständig durch den eingangs genannten mehrschichtigen Prophylaxeartikel und eine Zusammensetzung für die Bildung einer Gleitschicht des Prophylaxeartikels, wobei zumindest 90 % der Mikrokapseln als Singulärkapseln einen Durchmesser von weniger als 10 µm aufweisen, und wobei die Mikrokapseln sowohl als Singulärkapseln als auch als Agglomerate vorliegen, gelöst. Vorteilhaft dabei erweist sich, dass das Tastgefühl in den Endgliedern der Finger, insbesondere im Bereich der Fingerbeere vollständig erhalten bleibt. Zudem sind die Mikrokapseln durch die geringen Abmessungen weder während der Herstellung der Zusammensetzung oder der Produktion der Prophylaxeartikel noch vor dem bzw. beim Anziehen einem allzu großen Druck ausgesetzt und platzen daher nicht vorab und die darin enthaltenen Wirkstoffe werden daher nicht frühzeitig freigesetzt. Es ist vorgesehen, dass die Singulärkapseln Agglomerate bilden, wobei durch die Erhöhung der Oberflächenrauhigkeit an der Innenseite des Prophylaxeartikels die Anziehbarkeit und auch Nassschlüpfrigkeit verbessert werden können. Weiters erweist sich von Vorteil, dass die Agglomerate den Effekt der besseren Anziehbarkeit eines Prophylaxeartikels, der bereits durch sein Herstellungsverfahren eine gewisse Oberflächenrauhigkeit aufweisen kann, noch verstärken. Des weiteren erweist sich von Vorteil, dass durch die Oberflächenrauhigkeit kein vollflächiger Kontakt zwischen dem Prophylaxeartikel und der menschlichen Haut besteht und es dadurch zu einer verminderten Schweißbildung kommt bzw. der gebildete Schweiß leichter abtransportiert werden kann und somit der Tragekomfort für den Träger des Prophylaxeartikels erhöht wird. Durch die kleinere Berührungsfläche zwischen dem Prophylaxeartikel und der menschlichen Haut wird weiters die Verträglichkeit des Prophylaxeartikels gesteigert und die Häufigkeit des Auftretens von allergischen Reaktionen vermindert bzw. bei geeigneter Auswahl der Wirkstoffe zumindest annähernd zur Gänze vermieden. Von Vorteil erweist sich des weiteren auch, dass beim An- bzw. Überziehen des Prophylaxeartikels es zu einer Dehnung kommt und in der Folge dessen Wandstärke derart verringert wird, dass die Oberflächenrauhigkeit aufgrund der weiter hervortretenden Partikel verstärkt wird und das Anziehen zusätzlich erleichtert wird.

Durch den Farbstoff kann beispielsweise die Verteilung der Mikrokapseln in der Gleitschicht verfolgt und beim Aufplatzen der Mikrokapseln kann auch die Verteilung des Inhalts, also des Farb- aber auch des Wirkstoffs, visuell beobachtet werden.

Vorteilhafterweise beträgt der Durchmesser der Singulärkapseln einen Wert ausgewählt aus einem Bereich mit einer unteren Grenze von 0,1 µm, vorzugsweise 0,5 µm, insbesondere 1 µm, und einer oberen Grenze von 10 µm, vorzugsweise 9 µm, insbesondere 8 µm auf, wodurch eine einfache Verarbeitung der Mikrokapseln erzielt wird ohne dabei die Wirksamkeit derselben einzuschränken.

Der Durchmesser der Mikrokapseln beträgt weniger als 50 %, vorzugsweise 45 %, insbesondere 40 %, der Dicke der Gleitschicht, wodurch die Mikrokapseln, sofern sie randständig an der inneren Oberfläche der Gleitschicht angeordnet sind, teilweise über die Oberfläche der Gleitschicht vorstehen und somit der inneren Oberfläche des Prophylaxeartikels zusätzlich zu der beim Herstellungsprozess verursachten Unregelmäßigkeit eine Oberflächenstrukturierung verleihen. Weiters erweist sich als vorteilhaft, dass die Abmessungen der Singulärkapseln geringer als die Dicke der Gleitschicht sind, weil somit das Tastgefühl, insbesondere wenn sich Mikrokapseln im Bereich der Fingerspitzen befinden, aufrecht und die Taktilität trotz Tragens von z.B. Handschuhen vollständig erhalten bleibt. Vorteilhaft ist auch, dass die Haftung der Kapseln in der Gleitschicht höher ist, wenn die Abmessungen der Singulärkapsel geringer als die Dicke der Gleitschicht sind.

Von Vorteil erweist sich auch, dass der in den Mikrokapseln enthaltene Wirkstoff antibakterielle, antivirale, antitranspirante bzw. spermizide Eigenschaften enthalten sein kann. Dabei erweist sich von Vorteil, dass durch die antibakterielle bzw. antivirale Wirkung Infektionen viralen und bakteriellen Ursprungs bei Zerstörungen bzw. Verletzungen des Prophylaxeartikels vermieden werden können. Durch die antitranspirante Wirkung wird die Schweißbildung des Verwenders reduziert und dadurch der Tragekomfort des Prophylaxeartikels vergrößert. Durch die spermizide Wirkung der Wirkstoffe kann beispielsweise die kontrazeptive Wirkung bei Verletzungen bzw. Zerplatzen eines Kondoms dennoch aufrechterhalten werden.

Von Vorteil ist auch, dass der Wirkstoff und/oder Farbstoff bei einer Temperatur mit einer oberen Grenze von 42 °C, insbesondere 41 °C, vorzugsweise 40 °C, und einer unteren Grenze von 28 °C, insbesondere 32 °C, vorzugsweise 36 °C, löslich ist, wodurch bei physiologischen Umgebungstemperaturen, wie sie beim Tragen des Prophylaxeartikels, insbesondere Handschuhs, durch einen Menschen vorliegen, die Substanzen durch die vom Träger des Prophylaxeartikels produzierte bzw. abgegebene Wärme gelöst werden können und somit ihre optimale Wirkung entfalten können.

Die Mikrokapseln können integriert und/oder direkt angrenzend zur inneren Oberfläche der Gleitschicht in Richtung der Trägerschicht angeordnet sein, wodurch der von den Mikrokapseln freigegebene Wirkstoff bzw. Farbstoff in direktem Kontakt mit der Oberfläche, insbesondere Haut, des Verwenders treten kann und sich nicht erst einen Weg durch die Gleitschicht bahnen muss und somit verzögert am Wirkort austreten kann.

In einer Weiterbildung der Erfindung ist vorgesehen, dass der Anteil der Mikrokapseln in der Gleitschicht weniger als 1 Gew.-% beträgt, wodurch die ursprünglichen Eigenschaften der Gleitschicht, d.h. die verbesserte Anziehbarkeit, durch die Mikrokapseln nicht beeinträchtigt wird. Einerseits wird die Haftung der Gleitschicht, welche die Mikrokapseln mit umfasst, an der Trägerschicht bei geringer Kapselmenge besser, da eine Gleitschicht mit einer großen Menge an Mikrokapseln aufgrund der vielen Berührungspunkte der Mikrokapseln, die direkt den Gummi berühren und per se schlecht an diesem haften, sich nicht ausreichend mit der Trägerschicht verbindet, insbesondere haftet. Andererseits kann damit auch erzielt werden, dass die Gleitschicht des Prophylaxeartikels sehr kostengünstig hergestellt werden kann, ohne auf die durch den Wirkstoff bzw. Farbstoff verliehenen Eigenschaften der Mikrokapseln verzichten zu müssen.

Weiters ist vorgesehen, dass der Anteil der Mikrokapseln in der Gleitschicht aus einem Bereich mit einer unteren Grenze von 0,05 Gew.-%, vorzugsweise 0,08 Gew.-%, insbesondere 0,12 Gew.-%, und einer oberen Grenze von 0,95 Gew.-%, vorzugsweise 0,8 Gew.-%, insbesondere 0,75 Gew.-%, ausgewählt ist, wodurch einerseits die Gleitschicht kostengünstig hergestellt werden kann und andererseits auch aufgrund der geringen verwendeten Menge standardisierte Verfahren zur Herstellung bzw. Mischung der Gleitschicht nach wie vor verwendet werden können und es ist daher nicht erforderlich den Herstellungsprozess der Prophylaxeartikels auf die Verarbeitung von Mikrokapseln aufwändig umzustellen.

Durch den Zusammenschluss von 100 bis 10000 Singulärkapseln zu jeweils einem Agglomerat steht ein größeres Volumen des Wirk- und/oder Farbstoffs beim Zerplatzen, insbesondere an jenen Stellen zur Verfügung, die einem hohen Druck ausgesetzt sind, wie z.B. im Knöchelbereich. Vorwiegend diese Bereiche der Hand sind gefährdet Hautschäden zu erleiden und können somit gezielt geschützt werden.

Weiters erweist sich von Vorteil, dass die Agglomerate annähernd kugelförmig geformt sind und gegebenenfalls ein fußballähnliches Aussehen aufweisen, wodurch unter anderem die Gleiteigenschaften der Gleitschicht verbessert werden. Zudem wird durch den fußballähnlichen Aufbau der Agglomerate es ermöglicht eine maximale Anzahl von Mikrokapseln anzuordnen und somit die Wirk- bzw. Farbstoffkonzentration zu erhöhen.

In einer Weiterbildung ist vorgesehen, dass die Agglomerate hohl sind, wodurch sich ein größerer Umfang ergibt, somit die Unebenheiten an der Oberfläche der Gleitschicht vergrößert werden und dadurch die Gleiteigenschaften abermals verbessert werden, weil weniger direkte Kontaktfläche zwischen der inneren Oberfläche der Gleitschicht und der Hautoberfläche des Verwenders vorliegt.

Gemäß einer Ausführungsvariante können sich mehrere Agglomerate aneinanderlagern, wodurch eine hohe Konzentration des Wirk- und/oder Farbstoffs auf knappem Raum zur Verfügung steht.

Die Größe der Agglomerate ist vorzugsweise aus einem Bereich mit einer unteren Grenze von 5 µm, vorzugsweise 10 µm, insbesondere 15 µm, und einer oberen Grenze von 100 µm, vorzugsweise 80 µm, insbesondere 70 µm, ausgewählt, wodurch einerseits durch die erzielte Oberflächenrauhigkeit der Gleitschicht durch die Agglomerate die Anziehbarkeit und auch die Nassschlüpfrigkeit des Prophylaxeartikels, insbesondere Handschuhs, verbessert werden. Dadurch wird der Vorteil erreicht, dass im Vergleich mit herkömmlichen Handschuhen ohne diese Nassschlüpfrigkeit die Wirkstoffe und/oder Farbstoffe über einen längeren Zeitraum an die Haut abgegeben werden können und nicht sofort beim Anziehen des Handschuhs aufgrund der mechanischen Belastung, insbesondere Reibung, zur Gänze freigesetzt werden. Andererseits wird durch die Ansammlung vieler Singulärkapseln zu Agglomeraten eine hohe Menge des Wirkstoffs bzw. Farbstoffs an der mit Druck beaufschlagten Stelle, d.h. an jener Stelle, wo der Wirkstoff bzw. Farbstoff auch tatsächlich benötigt wird, freigesetzt.

In einer Weiterbildung ist vorgesehen, dass die Singulärkapseln und/oder Agglomerate eine Beschichtung aufweisen, wodurch ihnen besondere Eigenschaften, wie z.B. antistatische Eigenschaften, verbesserte Gleiteigenschaften, bessere Mischbarkeit, etc. verliehen werden können, die die Verarbeitung und auch den Tragekomfort des Prophylaxeartikels verbessern. Es können auch nur die Agglomerate eine Beschichtung aufweisen, wobei dadurch eine vordefinierbare Anzahl von Singulärkapseln in der Beschichtung zu einer Einheit, d.h. zu einem Agglomerat, zusammengefasst wird und somit eine definierte Menge des Wirkstoffs bzw. Farbstoffs freigesetzt werden kann.

Vorteilhaft erweist sich des weiteren, dass bei Verwendung des Prophylaxeartikels eine Änderung der Oberflächenstruktur der Gleitschicht erfolgt, wodurch bei Druckbeaufschlagung ein Platzen der Singulärkapseln bzw. der Agglomerate verursacht wird und in der Oberfläche der Gleitschicht Vertiefungen zurückbleiben, die eine Oberflächenstruktur hinterlassen, die unter anderem den Tragekomfort des Prophylaxeartikels verbessern.

In einer Weiterbildung können die Mikrokapseln mit einem Farbstoff gekennzeichnet sein, wodurch Auskunft über die Verteilung der Mikrokapseln in der Gleitschicht durch die visuelle Detektierbarkeit gegeben werden kann. Von Vorteil erweist sich dabei weiters, dass das Aufplatzen der Partikel unter Druckbelastung visuell beobachtet werden kann und dadurch für den Verwender des Prophylaxeartikels eine Gewährleistung der Freisetzung der Wirkstoffe und/oder Farbstoffe gegeben ist.

Weiters erweist sich von Vorteil, dass die Singulärkapseln und/oder Agglomerate wasserunlöslich sind, wodurch diese über ein Tauchverfahren aufgebracht werden können und die Wirkstoffe bzw. die Farbstoffe nicht bereits während der Herstellung des Prophylaxeartikels in den Tauchschritten bzw. anschließenden Waschschritten freigesetzt werden.

Gemäß einer alternativen Ausführungsvariante ist vorgesehen, dass die Singulärkapseln und/ oder Agglomerate wasserlöslich sind, wodurch diese auch in einem Sprühverfahren aufgebracht werden können und somit ein weiteres Herstellungsverfahren zur Herstellung des Prophylaxeartikels zur Verfügung steht. Weiters erweist sich dabei von Vorteil, dass bereits durch den Kontakt der Partikel mit z.B. Schweiß oder Sperma des Verwenders eine Freisetzung der Wirkstoffe und/oder Farbstoffe erfolgen kann und keine Druckbelastung zum Freisetzen der Wirkstoffe und/oder Farbstoffe notwendig ist, sodass eine Wirkstoffabgabe auch dann noch möglich ist, wenn der Verwender eine unpassende Größe des Prophylaxeartikels wählt.

Vorzugsweise sind die Mikrokapseln zumindest bereichsweise im Bereich des distalen Unterarms, Handgelenks, der Handwurzelknochen, der Mittelhandknochen und/oder der Grund-, Mittel- und Endglieder der Finger eines medizinischen Handschuhs angeordnet, wodurch insbesondere an jenen Stellen, welche bei Chirurgen bzw. medizinischen Personal besonders den Gefahrenstoffen, d.h. Mikroorganismen, exponiert sind bzw. jene Stellen, die mit dem Prophylaxeartikel bedeckt sind, mit entsprechenden Wirkstoffen bzw. Farbstoffen versorgt werden und dies zudem noch gezielt erfolgen kann, indem die Mikrokapseln vorwiegend an den jeweils stark beanspruchten Bereichen aufgebracht werden.

Gemäß einer Weiterbildung ist vorgesehen, dass die Mikrokapseln sowohl palmar als auch dorsal in zumindest einem Teilbereich des Prophylaxeartikels aufgebracht sind, wodurch nicht nur der erhöhte Feuchtigkeitsbedarf der dorsalen Handseite gedeckt werden kann, sondern auch die vermehrte Transpiration an der palmaren Seite vermieden werden kann.

Das Verfahren zur Herstellung des Prophylaxeartikels kann folgende Schritte umfassen: a) Tauchen einer Form in eine Koagulanslösung, b) Antrocknen, c) Tauchen in eine Elastomermischung, d) Antrocknen, e) Tauchen in eine Zusammensetzung mit Mikrokapseln (7) mit einem Durchmesser von weniger als 10 µm, f) Antrocknen, g) Auslaugen, h) Trocknen, i) Abziehen von der Form, j) Waschen, k) Chlorieren und l) Trocknen. Dadurch wird als Grundlage ein standardisiertes Verfahren eingesetzt, dass nur mehr um die erfindungsgemäßen Schritte erweitert werden muss und anschließend somit auch vollautomatisiert durchgeführt werden kann.

Die Konzentration der Mikrokapseln in der erfindungsgemäßen Zusammensetzung ist zwischen 0,05 % und 10 % ausgewählt, wodurch eine ausreichende Menge an Wirkstoffen dem Verwender des Prophylaxeartikels zur Verfügung gestellt wird, wobei allerdings auch eine kostenbewusste Herstellung des Prophylaxeartikels ermöglicht wird.

Werden weitere Stoffe wie zumindest ein Acrylat, carboxyliertes Butadien und oder Silicon, insbesondere selbstvernetzendes Silicon für die Zusammensetzung verwendet, können gewünschte Eigenschaften, wie höhere Elastizität oder bessere Verformbarkeit erzielt werden. Wird das Silicon bereits der Zusammensetzung beigemengt wird bei Ökonomisierung des Verfahrens zudem die Anziehbarkeit des Prophylaxeartikels verbessert. Der nachfolgende Schritt zum Aufbringen einer Siliconemulsion wird obsolet.

Der pH-Wert der Zusammensetzung ist annähernd neutral, wodurch eine zusätzliche Reizung, welche schon alleine durch das Tragen des Prophylaxeartikels hervorgerufen werden kann, unterbleibt.

Vorzugsweise ist der pH-Wert aus einem Bereich mit einer unteren Grenze von 5,5, vorzugsweise 6, insbesondere 6,5, und einer oberen Grenze von 8, vorzugsweise 7,5, insbesondere 7,2, ausgewählt, wodurch die bereits erwähnte zusätzliche Reizung der Haut des Trägers verhindert wird, weil der pH-Wert der Gleitschicht annähernd gleich dem der Haut des Trägers ist, d.h. im physiologischen Bereich, liegt.

Die Erfindung wird im Nachfolgenden anhand des in der Zeichnung dargestellten Ausführungsbeispiels näher erläutert.

Es zeigt:
- Fig. 1: einen Schnitt durch einen Prophylaxeartikel;
- Fig. 2: einen Schnitt durch die Gleitschicht eines erfindungsgemäßen Prophylaxeartikels.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen.

Die vorliegende Erfindung beschreibt einen mehrschichtigen Prophylaxeartikel.

Ein Prophylaxeartikel im Sinne der Erfindung ist ein Hanschuh, insbesondere einen medizinischen Handschuh, oder ein Kondom, welcher oder welches aus mehreren Schichten aufgebaut ist.

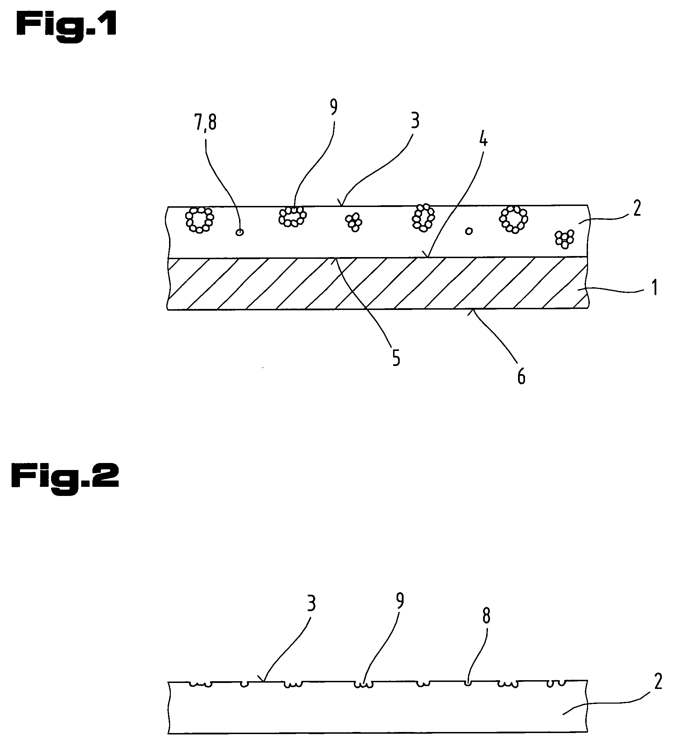

Wie in Fig. 1 dargestellt besteht der Prophylaxeartikel zumindest aus einer elastomeren Trägerschicht 1 und zumindest bereichsweise einer Gleitschicht 2. Beide Schichten weisen eine innere 3, 5 und eine äußere Oberfläche 4, 6 auf, wobei die äußere Oberfläche 4 der Gleitschicht 2 der inneren Oberfläche 5 der Trägerschicht 1 zugewandt ist. Die äußere Oberfläche 4, 6 der beiden Schichten ist jeweils dem Verwender des Prophylaxeartikels abgewandt und die innere Oberfläche 3, 5 der beiden Schichten ist jeweils der Oberfläche, d.h. der Hautoberfläche des Trägers, zugewandt.

Erfindungsgemäß sind in der Gleitschicht 2 Mikrokapseln 7 enthalten. Die Mikrokapseln 7 liegen sowohl als Singulärkapseln 8 als auch als Agglomerate 9 vor.

Der Durchmesser der Singulärkapsel 8 beträgt weniger als 10 µm. Im Speziellen weist der Durchmesser einer Singulärkapsel 8 einen Wert ausgewählt aus einem Bereich mit einer unteren Grenze von 0,1 µm, vorzugsweise 0,5 µm, insbesondere 1 µm, und einer oberen Grenze von 10 µm, vorzugsweise 9 µm, insbesondere 8 µm, auf. Durch die geringen Abmessungen des Durchmessers der Singulärkapseln 8 kommt es zu keiner bzw. nur einer geringfügigen Änderung der Struktur der inneren Oberfläche 3 der Gleitschicht 2.

Die Dicke der Gleitschicht ist so ausgewählt, dass der Durchmesser der Mikrokapseln 7 weniger als 50 %, vorzugsweise 45 %, insbesondere 40 %, der Dicke der Gleitschicht 2 beträgt. Sind mehrere Mikrokapseln 7 zu Agglomeraten 9 verbunden, so weisen auch die Agglomerate 9 einen geringeren Durchmesser als die Dicke der Gleitschicht 2 auf. Die Agglomerate 9 weisen einen Durchmesser von 5 µm bis maximal 100 µm auf und bestehen aus einer Vielzahl von Singulärkapseln 8. Die Anzahl der Singulärkapsel 8, welche Agglomerate 9 bilden, kann unterschiedlich sein, weil diese von vielen Faktoren abhängig ist, wie beispielsweise der Beschaffenheit der in den Mikrokapseln 7 zu verpackenden Substanz. Erfindungsgemäß ist die Anzahl der Singulärkapseln 8, welche Agglomerate 9 bilden aus einem Bereich mit einer unteren Grenze von 100, insbesondere 200, vorzugsweise 500, und einer oberen Grenze von 10.000, insbesondere 5.000, vorzugsweise 1.000, ausgewählt.

Die Hülle der Mikrokapseln 7 besteht vorzugsweise aus einem Formaldehydharz und ist vorzugsweise nicht wasserlöslich. In den Mikrokapseln 7, die als Singulärkapsel 8 oder als Agglomerat 9 vorliegen, ist zumindest ein Wirkstoff und/oder Farbstoff eingeschlossen, der bei Bedarf, insbesondere durch Druckbeaufschlagung oder durch Reibung, freigesetzt werden kann. Die Wirkstoffe können dem Prophylaxeartikel antibakterielle, antivirale, antitranspirante und/ oder spermizide Eigenschaften verleihen. So können beispielsweise pflegende und hautfreundliche Substanzen, wie z.B. Glycerylstearat, Glyceryllaurat, Octylstearat, Octylpalmitat, Tocopherylnicotinat, PEG, Kollagen. Fruchtsäuren, Fettsäuren, Quercetin, Aloe Vera, etc., Duftstoffe, wie Vanille, etc. in den Mikrokapseln 7 verpackt sein.

In einer bevorzugten Ausführungsform sind die Mikrokapseln 7 geschlossen. In einer alternativen Ausführungsvariante ist es auch möglich, dass die Oberfläche der Mikrokapseln 7, z.B. porös, ausgebildet ist. Die verpackten Substanzen können wasserlöslich sein und somit durch den Kontakt mit der Hautoberfläche und somit dem Schweiß gelöst und aktiviert werden.

Beim Tragen des Prophylaxeartikels bzw. beim Überziehen des Prophylaxeartikels platzen die Singulärkapseln 8 oder Agglomerate 9 und setzen den darin enthaltenen Wirkstoff bzw. Farbstoff frei. Es kann dabei zu einer Änderung der Oberflächenstruktur der inneren Oberfläche 3 der Gleitschicht 2 kommen, wie dies in Fig. 2 dargestellt ist.

Bei einer Temperatur zwischen 28 °C und 42 °C ist der Wirkstoff und/oder Farbstoff löslich, wobei dies die physiologische Temperatur und deren Randbereiche eines Menschen wiederspiegelt und sich somit die Wirkung des Wirkstoffes bzw. des Farbstoffes in diesem Temperaturbereich voll entfalten kann.

Die Mikrokapseln 7, unabhängig davon ob sie als Singulärkapsel 8 oder als Agglomerat 9 vorliegen, sind entweder an der inneren Oberfläche 3 der Gleitschicht 2 oder direkt unterhalb der inneren Oberfläche 3 der Gleitschicht 2 in Richtung der äußeren Oberfläche 4 angeordnet, um rasch die in den Mikrokapseln verpackte Wirksubstanz am Ort des Geschehens freisetzen zu können und somit die erforderliche Wirkung bzw. Färbung zu bewirken.

Unabhängig von der Anordnung der Agglomerate 9 im Prophylaxeartikel können sich auch mehrere Agglomerate aneinanderlagern, wodurch Knäuel von Agglomeraten 9 entstehen.

Der Anteil der Mikrokapseln 7 in der Gleitschicht 2 bzw. der Zusammensetzung zur Herstellung der Gleitschicht 2 beträgt weniger als 1 Gew.-% und ist vorzugsweise aus einem Bereich mit einer unteren Grenze von 0,05 Gew.-%, vorzugsweise 0,08 Gew.-%, insbesondere 0,12 Gew.-%, und einer oberen Grenze von 0,95 Gew.-%, vorzugsweise 0,8 Gew.-%, insbesondere 0,75 Gew.-%, ausgewählt.

Sowohl die Singulärkapseln 8 als auch die Agglomerate 9 können eine Beschichtung aufweisen, wobei durch die Auswahl der entsprechenden Beschichtung den Mikrokapseln zusätzliche gewünschte Eigenschaften verliehen werden können.

Im Aussehen sind die erfindungsgemäßen Agglomerate 9 vorzugsweise kugelförmig ausgebildet, insbesondere ähneln sie der Form eines Fußballs und sind hohl.

Die Mikrokapseln 7 können neben dem Farbstoff, den sie in ihrem Inneren enthalten, auch an der äußeren Oberfläche einen Farbstoff aufweisen, um deren Verteilung in der Gleitschicht 2 besser beobachten zu können.

Je nach Bedarf können die Singulärkapseln 8 und/oder Agglomerate 9 wasserlöslich oder wasserunlöslich sein. Sind sie wasserunlöslich können nicht bereits während des Herstellungsprozesses die Wirk- und/oder Farbstoffe freigesetzt werden. Bei wasserlöslichen Partikeln kann auch durch die Einwirkung von Schweiß bewirkt werden, dass sich die Singulärkapseln 8 und/ oder Agglomerate 9 lösen und somit der Wirk- und/oder Farbstoff freigesetzt wird.

Ist der Prophylaxeartikel in Form eines medizinischen Handschuhs ausgebildet, so sind die Mikrokapseln 7 entweder als Singulärkapsel 8 oder als Agglomerat 9 überall in der Gleitschicht verteilt bzw. können je nach Bedarf bereichsweise im Bereich des distalen Unterarms, Handgelenks, der Handwurzelknochen, der Mittelhandknochen und/oder der Grund-, Mittel- oder Endglieder der Finger angeordnet sein.

Bei medizinischen Handschuhen, welche vorzugsweise von Mikrochirurgen verwendet werden, die maximal mit der Oberfläche des Patienten, d.h. dem potentiell infektiösen Agens, in Berührung kommen, ist eine Beimengung der mit Wirkstoff versehenen Mikrokapseln 7 beispielsweise im Bereich der Fingerspitzen ausreichend. Kommt es allerdings zu Stichverletzungen kann sich die Anordnung der Mikrokapseln 7 auch in anderen Bereichen als vorteilhaft erweisen.

Wie bereits vorab erwähnt, kann je nach Beanspruchung die Anordnung der Mikrokapseln 7, sowohl palmar als auch dorsal erfolgen.

Bei dem Verfahren, welches zur Herstellung des erfindungsgemäßen Prophylaxeartikels verwendet wird, werden zumindest 90 % der Mikrokapseln 7 als Singulärkapseln 8 mit einem Durchmesser von weniger als 10 µm verwendet.

Es ist seit langem in der Fachwelt bekannt, dass, um reproduzierbare Schichten der Trägerschicht 1 aus zumindest einem Elastomer auf einer Form herstellen zu können, auf die Form, beispielsweise auf eine mit entsprechenden Aufrauungen oder mit einer glatten Oberfläche versehene Keramikform, ein Koagulant aufgebracht wird. Dazu wird üblicherweise die Form in ein Becken bzw. einen Behälter, in welchem der Koagulant in flüssiger Form vorhanden ist, eingetaucht. Dieser Koagulant kann jede aus dem Stand der Technik bekannte Zusammensetzung aufweisen, wie beispielsweise Alkohollösungen von Kalziumsalzen oder dgl. Der Koagulant kann auch ein Trennmittel enthalten, wie beispielsweise Talkum oder Kalziumcarbonat, das, wenn es säurelöslich ist, bei nachfolgenden Säurebehandlungen aus den Oberflächenschichten herausgelöst werden kann, sodass ein sog. puderfreier Handschuh entsteht. Anschließend wird der Koagulant getrocknet.

Daraufhin wird die Form mit dem bevorzugt getrockneten Koagulant in einen Behälter, in dem das Elastomer als Dispersion bzw. Flüssigkeit vorrätig gehalten wird, eingetaucht. Um die Schichtdicke zu erhöhen, kann nach einem kurzen Antrocknen der Latex-Schicht die Form mehrmals eingetaucht werden.

Durch die chemische Reaktion des Elastomers mit dem Koagulant härtet das flüssig aufgetragene Elastomer aus. Bevorzugt wird unmittelbar nach dem Aufbringen des Elstomers auf die Form diese mit Heißluft kurz angetrocknet, sodass die Oberflächen der Trägerschicht 1 fest bzw. geliert werden, wobei diese beispielsweise in einem Wärmeofen oder einem Behälter, in dem Warmluft mit einer Temperatur zwischen 70°C und 140°C hindurchgeführt wird, 15 sec. bis 60 sec. getrocknet werden.

Nach der Zwischentrocknung wird auf die Trägerschicht 1 zumindest in einem Teilbereich der Oberfläche der Trägerschicht 1, vorzugsweise auf die gesamte Fläche, die Gleitschicht 2 aus polymeren Material enthaltend Mikrokapseln 7 durch Tauchen oder Sprühen in einem oder mehreren Schritten auf die angetrocknete Trägerschicht 1 aufgetragen. Die Mikrokapseln 7 können bereits vorher dispergiert werden, z.B. in einem Acryllack, oder direkt aus dem wässrigen Slurry des Herstellungsprozess ohne Trocknungsschritt beigemengt werden. Der Durchmesser der Mikrokapseln 7 kann aus einem Bereich mit einer oberen Grenze von 10 µm, insbesondere 9 µm, vorzugsweise 8 µm und einer unteren Grenze von 0,1 µm, vorzugsweise 0,5 µm, insbesondere 1 µm, ausgewählt sein. Der Durchmesser der Mikrokapseln 7 kann auch einen Wert annehmen ausgewählt aus einem Bereich mit einer oberen Grenze von 7 µm, vorzugsweise 6 µm, insbesondere 5 µm, und einer unteren Grenze von 1,2 µm, vorzugsweise 1,5 µm, insbesondere 2 µm.

Die Schichtdicke der Gleitschicht 2 kann entsprechend den unterschiedlichen Erfordernissen, insbesondere der danach gewünschten Rautiefe und der Größe der Partikel, festgelegt werden und kann auch solange aufgetragen werden, bis diese Dicke erreicht ist.

Die Gleitschicht 2 kann durch ein heterogenes Gemisch aus zumindest einem polymeren Material, wie beispielsweise einer wässrigen Polyurethandispersion, und Partikeln, insbesondere Mikrokapseln 7, Liposomen, etc., gebildet werden.

Als polymeres Material kann je nach Einsatzzweck ein Polyacrylat, ein Polysiloxan, ein Poly(meth)acrylat, ein carboxyliertes Styrol-Butadien Copolymer, ein Polyvinylpyrollidon, ein kationisches Polyurethan und Mischungen daraus, z.B. mit einem Molekulargewicht von mindestens 100.000 Da, ebenso verwendet werden, wie die nichtionischen oder anionischen Ausführungen dieser vorgenannten Materialien. Das heterogene Gemisch, insbesondere die wässrige Dispersion aus den polymeren Materialien und Mikrokasplen 7 und beliebige Mischungen davon, bildet Schichten bzw. Filme mit guten mechanischen Grundeigenschaften und weisen diese vorzugsweise ähnliche Dehnungseigenschaften auf wie die Trägerschicht 1. Die Konzentration der Mikrokasplen 7 im heterogenen Gemisch kann aus einem Bereich mit einer unteren Grenze von 0,05 Gew.-%, insbesondere 0,08 Gew.-%, vorzugsweise 0,12 Gew.-%, und einer oberen Grenze von 1 Gew.-%, vorzugsweise 0,95 Gew.-%, insbesondere 0,8 Gew.-%, ausgewählt sein. Vorzugsweise ist die Konzentration der Mikrokasplen 7 im heterogenen Gemisch aus einem Bereich mit einer unteren Grenze von 0,15 Gew.-%, vorzugsweise 0,2 Gew.-%, insbesondere 0,3 Gew.-%, und einer oberen Grenze von 0,6 Gew.-%, vorzugsweise 0,5 Gew.-%, insbesondere 0,4 Gew.-%, ausgewählt.

Nach dem Herausheben des Formträgers mit den Tauchformen aus dem heterogenen Gemisch aus polymerem Material und Mikrokapseln 7 zur Herstellung der Gleitschicht 2, wird die aufgebrachte Gleitschicht 2 getrocknet, vorzugsweise mit Heißluft, beispielsweise bei einer Temperatur zwischen 70°C und 140°C, bevorzugt 90°C bis 110°C, über eine Dauer von 15 sec. bis 60 sec. angetrocknet. Bevorzugt werden die Temperatur und die Zeitdauer der Heißluftbehandlung so abgestimmt, dass die Oberfläche der Gleitschicht 2 in einen gelartigen bzw. festen Zustand übergeht.

Unmittelbar darauf werden die Tauchformen mit den darauf befindlichen Rohteilen der Prophylaxeartikel , insbesondere Handschuhe, in ein weiteres Tauchbecken eingetaucht, in welchem die Gleitschicht 2 mit Wasser, vorzugsweise heißem Wasser mit einer Temperatur zwischen 40°C und 95°C, bevorzugt 70°C bis 90°C, besprüht oder gespült wird. Dieser Vorgang wird in der Fachsprache "leaching" bezeichnet.

Durch diese Behandlung mit heißem Wasser wird die chemische Reaktion in der Gleitschicht 2 und der Trägerschicht 1 eingeleitet bzw. unterstützt, sodass es zum Einleiten oder zur vollständigen Koagulation in dieser Schicht kommt.

Nach dem genannten Trocknungsvorgang werden die Prophylaxeartikel von den Formen abgezogen, gegebenenfalls "offline" gewaschen, chloriert um die Oberflächenstruktur zu verändern und abermals getrocknet.

In einer alternativen Ausführungsform können die Mikrokapseln 7 auch auf die Gleitschicht 2 nach deren Anbindung mit der Trägerschicht 1, z.B. durch Sprüchen, aufgebracht werden.

Die Gleitschicht wird von einer Zusammensetzung gebildet, die aus einem Gemisch umfassend Wasser, Polyurethan und Mikrokapseln 7 besteht, wobei 90 % der Mikrokapseln 7 einen Durchmesser von weniger als 10 µm aufweisen.

Die Zusammensetzung kann weiters zumindest ein Acrylat, carboxyliertes Butadien und Silicon, insbesondere selbstvernetzendes Silikon enthalten.

Der pH-Wert der Zusammensetzung ist annähernd neutral und vorzugsweise aus einem Bereich mit einer unteren Grenze von 5,5 und einer oberen Grenze von 8 ausgewählt, um den physiologischen Bedingungen der Hautoberfläche so nahe wie möglich zu kommen.

Bezüglich des detaillierten Aufbaus des Handschuhs, der verwendeten Materialien, dem Herstellungsverfahren und der zu verpackenden Wirkstoffe in den Mikrokapseln 7 sei zusätzlich auf die österreichische Offenlegungsschrift mit dem Aktenzeichen A 786/2003 der Anmelderin verwiesen.

Die Ausführungsbeispiele zeigen mögliche Ausführungsvarianten des Prophylaxeartikels, wobei an dieser Stelle bemerkt sei, dass auch diverse Kombinationen der einzelnen Ausführungsvarianten untereinander möglich sind.

Der Ordnung halber sei abschließend darauf hingewiesen, dass zum besseren Verständnis des Aufbaus des Prophylaxeartikels dieser bzw. dessen Bestandteile teilweise unmaßstäblich und/oder vergrößert und/oder verkleinert dargestellt wurden.

### Bezugszeichenaufstellung

- 1: Trägerschicht
- 2: Gleitschicht
- 3: innere Oberfläche
- 4: äußere Oberfläche
- 5: innere Oberfläche

- 6: äußere Oberfläche
- 7: Mikrokapsel
- 8: Singulärkapsel
- 9: Agglomerat

## Patentansprüche

1. Mehrschichtiger Prophylaxeartikel in Form eines Handschuhs, insbesondere eines medizinischen Handschuhs, oder Kondoms aus zumindest einer elastomeren Träger- (1) und zumindest bereichsweise einer Gleitschicht (2) mit jeweils einer inneren (3, 5) und einer äußeren Oberfläche (4, 6), wobei die äußere Oberfläche (4) der Gleitschicht (2) der inneren Oberfläche (5) der Trägerschicht (1) zugewandt ist, und in der Gleitschicht (2) Mikrokapseln (7) enthalten sind, die zumindest einen Wirkstoff und/oder Farbstoff enthalten, **dadurch gekennzeichnet, dass** zumindest 90 % der Mikrokapseln (7) als Singulärkapseln (8) einen Durchmesser von weniger als 10 µm aufweisen, und wobei die Mikrokapseln (7) sowohl als Singulärkapseln (8) als auch als Agglomerate (9) vorliegen.

2. Prophylaxeartikel nach Anspruch 1, **dadurch gekennzeichnet, dass** die Mikrokapseln (7) als Singulärkapseln (8) einen Durchmesser ausgewählt aus einem Bereich mit einer unteren Grenze von 0,1 µm, vorzugsweise 0,5 µm, insbesondere 1 µm, und einer oberen Grenze von 10 µm, vorzugsweise 9 µm, insbesondere 8 µm, aufweisen.

3. Prophylaxeartikel nach Anspruch 1 oder 2, **dadurch gekennzeichnet, dass** der in den Mikrokapseln (7) enthaltene Wirkstoff ein antibakterieller, antiviraler, antitranspiranter bzw. spermizider Wirkstoff ist.

4. Prophylaxeartikel nach einem der Ansprüche 1 bis 3, **dadurch gekennzeichnet, dass** der Wirkstoff und/ oder Farbstoff bei einer Temperatur mit einer oberen Grenze von 42°C, insbesondere 41°C, vorzugsweise 40°C, und einer unteren Grenze von 28°C, insbesondere 32°C, vorzugsweise 36°C, löslich ist.

5. Prophylaxeartikel nach einem der Ansprüche 1 bis 4, **dadurch gekennzeichnet, dass** der Anteil der Mikrokapseln (7) in der Gleitschicht (2) weniger als 1 Gew.-% beträgt.

6. Prophylaxeartikel nach Anspruch 5, **dadurch gekennzeichnet, dass** der Anteil der Mikrokapseln (7) in der Gleitschicht (2) aus einem Bereich mit einer unteren Grenze von 0,05 Gew.-%, vorzugsweise 0,08 Gew.-%, insbesondere 0,1 Gew.-%, und einer oberen Grenze von 0,95 Gew.-%, vorzugsweise 0,8 Gew.-%, insbesondere 0,75 Gew.-%, ausgewählt ist.

7. Prophylaxeartikel nach einem der Ansprüche 1 bis 6, **dadurch gekennzeichnet, dass** eine definierte Anzahl von Singulärkapseln (8) mit einer unteren Grenze von 100, insbesondere 200, vorzugsweise 500, und einer oberen Grenze von 10.000, insbesondere 5.000, vorzugsweise 1.000, jeweils ein Agglomerat (9) bilden.

8. Prophylaxeartikel nach einem der Ansprüche 1 bis 7, **dadurch gekennzeichnet, dass** die Agglomerate (9) annähernd kugelförmig, gegebenenfalls fußballähnlich geformt, sind.

9. Prophylaxeartikel nach einem der Ansprüche 1 bis 8, **dadurch gekennzeichnet, dass** die Agglomerate (9) hohl sind.

10. Prophylaxeartikel nach einem der Ansprüche 1 bis 9, **dadurch gekennzeichnet, dass** sich mehrere Agglomerate (9) aneinanderlagern.

11. Prophylaxeartikel nach einem der Ansprüche 1 bis 10, **dadurch gekennzeichnet, dass** die Agglomerate (9) eine Größe/Durchmesser ausgewählt aus einem Bereich mit einer unteren Grenze von 5 µm, vorzugsweise 10 µm, insbesondere 15 µm, und einer oberen Grenze von 100 µm, vorzugsweise 80 µm, insbesondere 70 µm, aufweisen.

12. Prophylaxeartikel nach einem der Ansprüche 1 bis 11, **dadurch gekennzeichnet, dass** der Durchmesser der Singulärkapseln (8) und/oder Agglomerate (9) weniger als 50 %, vorzugsweise 45 %, insbesondere 40%, der Dicke der Gleitschicht beträgt.

13. Prophylaxeartikel nach einem der Ansprüche 1 bis 12, **dadurch gekennzeichnet, dass** die Mikrokapseln (7) und/oder Agglomerate (9) integriert in die innere Oberfläche (3) und/ oder direkt angrenzend zur inneren Oberfläche (3) der Gleitschicht (2) in Richtung der Trägerschicht (1) angeordnet sind.

14. Prophylaxeartikel nach einem der Ansprüche 1 bis 13, **dadurch gekennzeichnet, dass** die Singulärkapseln (8) und/oder Agglomerate (9) eine Beschichtung aufweisen.

15. Prophylaxeartikel nach einem der Ansprüche 1 bis 14, **dadurch gekennzeichnet, dass** die Singulärkapseln (8) und/oder Agglomerate (9) mit einem Farbstoff gekennzeichnet sind.

16. Prophylaxeartikel nach einem der Ansprüche 1 bis 15, **dadurch gekennzeichnet, dass** die Singulärkapseln (8) und/oder Agglomerate (9) wasserunlöslich sind.

17. Prophylaxeartikel nach einem der Ansprüche 1 bis 15, **dadurch gekennzeichnet, dass** die Singulärkapseln (8) und/oder Agglomerate (9) wasserlöslich sind.

18. Prophylaxeartikel nach einem der Ansprüche 1 bis 17, **dadurch gekennzeichnet, dass** die Singulärkapseln (8) und/oder Agglomerate (9) zumindest bereichsweise im Bereich des distalen Unterarms, Handgelenks, der Handwurzelknochen, der Mittelhandknochen und/ oder der Grund-, Mittel- und Endglieder der Finger eines medizinischen Handschuhs angeordnet sind.

19. Prophylaxeartikel nach einem der Ansprüche 1 bis 18, **dadurch gekennzeichnet, dass** die Singulärkapseln (8) und/oder Agglomerate (9) zumindest bereichsweise palmar als auch dorsal angeordnet sind.

20. Prophylaxeartikel nach einem der Ansprüche 1 bis 19, **dadurch gekennzeichnet, dass** bei Verwendung eine Änderung der Oberflächenstruktur der Gleitschicht (2) erfolgt.

21. Zusammensetzung für die Bildung einer Gleitschicht (2) eines Prophylaxeartikels in Form eines Handschuhs oder Kondoms, umfassend ein Gemisch aus Wasser, Polyurethan und Mikrokapseln (7), die zumindest einen Wirkstoff und/oder Farbstoff enthalten, **dadurch gekennzeichnet, dass** zumindest 90 % der Mikrokapseln (7) einen Durchmesser von weniger als 10 µm aufweisen und sowohl als Singulärkapseln (8) als auch als Agglomerate (9) vorliegen.

22. Zusammensetzung nach Anspruch 21, **dadurch gekennzeichnet, dass** die Konzentration der Mikrokapseln (7) aus einem Bereich mit einer unteren Grenze von 0,01 %, vorzugsweise 0,1 %, insbesondere 0,5 %, und einer oberen Grenze von 10 %, vorzugsweise 5 %, insbesondere 1 %, ausgewählt ist.

23. Zusammensetzung nach Anspruch 21 oder 22, **dadurch gekennzeichnet, dass** Acrylat und/oder carboxyliertes Butadien und/oder Silicon enthalten sind.

24. Zusammensetzung nach Anspruch 23, **dadurch gekennzeichnet, dass** das Silicon selbstvernetzende Eigenschaften aufweist.

25. Zusammensetzung nach Anspruch 21 bis 24, **dadurch gekennzeichnet, dass** der pH-Wert annähernd neutral ist.

26. Zusammensetzung nach Anspruch 25, **dadurch gekennzeichnet, dass** der pH-Wert aus einem Bereich mit einer unteren Grenze von 5,5, vorzugsweise 6, insbesondere 6,5, und einer oberen Grenze von 8, vorzugsweise 7,5, insbesondere 7,2, ausgewählt ist.

27. Verwendung der Zusammensetzung nach einem der Ansprüche 21 bis 26 als Gleitschicht (2) für einen Prophylaxeartikel in Form eines Handschuhs, insbesondere medizinischen Handschuhs, oder Kondoms.

## Claims

1. Multi-layered prophylaxis item in the form of a glove, particularly a medical glove, or condom, comprising at least one elastomeric base layer (1) and at least one anti-friction layer (2) in some areas, each having an internal surface (3, 5) and an external surface (4, 6), wherein the external surface (4) of the anti-friction layer (2) faces the internal surface (5) of the base layer (1), and microcapsules (7) containing at least one active substance and/or colouring agent are contained in the anti-friction layer (2), **characterised in that** at least 90% of the microcapsules (7) as singular capsules (8) have a diameter of less than 10 µm, and wherein the microcapsules (7) are present both as singular capsules (8) and as agglomerates (9).

2. Prophylaxis item according to claim 1, **characterised in that** the microcapsules (7) as singular capsules (8) have a diameter selected from a range with a lower limit of 0.1 µm, preferably 0.5 µm, particularly 1 µm, and an upper limit of 10 µm, preferably 9 µm, particularly 8 µm.

3. Prophylaxis item according to claim 1 or 2, **characterised in that** the active substance contained in the microcapsules (7) is an antibacterial, antiviral, antiperspirant or spermicidal substance.

4. Prophylaxis item according to any one of claims 1 to 3, **characterised in that** the active substance and/or colouring agent is soluble at a temperature with an upper limit of 42 °C, particularly 41 °C, preferably 40 °C, and a lower limit of 28 °C, particularly 32 °C, preferably 36 °C.

5. Prophylaxis item according to any one of claims 1 to 4, **characterised in that** the percentage of microcapsules (7) in the anti-friction layer (2) is less than 1% by weight.

6. Prophylaxis item according to claim 5, **characterised in that** the percentage of microcapsules (7) in the anti-friction layer (2) is selected from a range having a lower limit of 0.05% by weight, preferably 0.08% by weight, particularly 0.1% by weight, and an upper limit of 0.95% by weight, preferably 0.8% by weight, particularly 0.75% by weight.

7. Prophylaxis item according to any one of claims 1 to 6, **characterised in that** a defined number of singular capsules (8) with a lower limit of 100, particularly 200, preferably 500, and an upper limit of 10,000, particularly 5,000, preferably 1,000, each form an agglomerate (9).

8. Prophylaxis item according to any one of claims 1 to 7, **characterised in that** the agglomerates (9) are approximately spherical, optionally football-shaped.

9. Prophylaxis item according to any one of claims 1 to 8, **characterised in that** the agglomerates (9) are hollow.

10. Prophylaxis item according to any one of claims 1 to 9, **characterised in that** several agglomerates (9) are arranged side by side.

11. Prophylaxis item according to any one of claims 1 to 10, **characterised in that** the agglomerates (9) have a size/diameter selected from a range with a lower limit of 5 µm, preferably 10 µm, particularly 15 µm, and an upper limit of 100 µm, preferably 80 µm, particularly 70 µm.

12. Prophylaxis item according to any one of claims 1 to 11, **characterised in that** the diameter of the singular capsules (8) and/or agglomerates (9) is less than 50%, preferably less than 45%, particularly less than 40%, of the thickness of the anti-friction layer.

13. Prophylaxis item according to any one of claims 1 to 12, **characterised in that** the microcapsules (7) and/or agglomerates (9) are integrated in the internal surface (3) and/or directly adjacent to the internal surface (3) of the anti-friction layer (2) in the direction of the base layer (1).

14. Prophylaxis item according to any one of claims 1 to 13, **characterised in that** the singular capsules (8) and/or agglomerates (9) have a coating.

15. Prophylaxis item according to any one of claims 1 to 14, **characterised in that** the singular capsules (8) and/or agglomerates (9) are marked with a colouring agent.

16. Prophylaxis item according to any one of claims 1 to 15, **characterised in that** the singular capsules (8) and/or agglomerates (9) are insoluble in water.

17. Prophylaxis item according to any one of claims 1 to 15, **characterised in that** the singular capsules (8) and/or agglomerates (9) are soluble in water.

18. Prophylaxis item according to any one of claims 1 to 17, **characterised in that** the singular capsules (8) and/or agglomerates (9) are arranged at least in areas of the region of the distal forearm, the wrist, the carpal bones, the metacarpal bones and/or the base, middle and terminal phalanges of the fingers of a medical glove.

19. Prophylaxis item according to any one of claims 1 to 18, **characterised in that** the singular capsules (8) and/or agglomerates (9) are arranged at least in areas of the palmar and dorsal regions.

20. Prophylaxis item according to any one of claims 1 to 19, **characterised in that** a change occurs in the surface structure of the anti-friction surface (2) during use.

21. Composition for forming an anti-friction surface (2) of a prophylaxis item in the form of a medical glove or condom, comprising a mixture of water, polyurethane and microcapsules (7) which contain at least one active substance and/or colouring agent, **characterised in that** at least 90% of the microcapsules (7) have a diameter of less than 10 µm and are present both as singular capsules (8) and as agglomerates (9).

22. Composition according to claim 21, **characterised in that** the concentration of microcapsules (7) is selected from a range with a lower limit of 0.01%, preferably 0.1%, particularly 0.5%, and an upper limit of 10%, preferably 5%, particularly 1%.

23. Composition according to claim 21 or 22, **characterised in that** acrylate and/or carboxylated butadiene and/or silicone are contained.

24. Composition according to claim 23, **characterised in that** the silicone has self-crosslinking properties.

25. Composition according to any one of claims 21 to 24, **characterised in that** the pH value is approximately neutral.

26. Composition according to claim 25, **characterised in that** the pH value is selected from a range with a lower limit of 5.5, preferably 6, particularly 6.5, and an upper limit of 8, preferably 7.5, particularly 7,2.

27. Use of the composition according to any one of claims 21 to 26 as an anti-friction layer (2) for a prophylaxis item in the form of a glove, particularly a medical glove, or a condom.

## Revendications

1. Article de prophylaxie multicouche sous la forme d'un gant, en particulier un gant médical, ou d'un préservatif, comprenant au moins un support en élastomère (1) et au moins par endroits une couche de glissement (2) ayant chacune une surface intérieure (3, 5) et une surface extérieure (4, 6), dans lequel la surface extérieure (4) de la couche de glissement (2) est tournée vers la surface intérieure (5) de la couche de support (1), et des microcapsules (7) sont présentes dans la couche de glissement (2), comprenant au moins une principe actif et/ou un colorant, **caractérisé en ce qu'**au moins 90 % des microcapsules (7), en tant que capsules individuelles (8), présentent un diamètre inférieur à 10 µm, et dans lequel les microcapsules (7) sont présentes à la fois sous la forme de capsules individuelles (8) ainsi que sous la forme d'agglomérats (9).

2. Article de prophylaxie selon la revendication 1, **caractérisé en ce que** les microcapsules (7) sous la forme de capsules individuelles (8) présentent un diamètre choisi dans une plage avec une limite inférieure de 0,1 µm, de préférence 0,5 µm, en particulier de 1 µm, et une limite supérieure de 10 µm, de préférence de 9 µm, en particulier de 8 µm.

3. Article de prophylaxie selon la revendication 1 ou 2, **caractérisé en ce que** dans les microcapsules (7), le principe actif présent est un principe actif antibactérien, antiviral, anti-transpiration et spermicide.

4. Article de prophylaxie selon l'une quelconque des revendications 1 à 3, **caractérisé en ce que** le principe actif et/ou un colorant est soluble à une température ayant une limite supérieure de 42°C, en particulier de 41°C, de préférence de 40°C, et une limite inférieure de 28°C, en particulier 32°C, de préférence 36°C.

5. Article de prophylaxie selon l'une quelconque des revendications 1 à 4, **caractérisé en ce que** la proportion des microcapsules (7) dans la couche de glissement (2) est inférieure à 1 % en poids.

6. Article de prophylaxie selon la revendication 5, **caractérisé en ce que** la proportion des microcapsules (7) dans la couche de glissement (2) est choisie dans une plage avec une limite inférieure de 0,05 % en poids, de préférence de 0,08 % en poids, en particulier de 0,1 % en poids, et une limite supérieure de 0,95 % en poids, de préférence de 0,8 % en poids, en particulier de 0,75 % en poids.

7. Article de prophylaxie selon l'une quelconque des revendications 1 à 6, **caractérisé en ce qu'**un nombre défini de capsules individuelles (8) forment respectivement un agglomérat (9) avec une limite inférieure de 100, en particulier de 200, de préférence de 500, et une limite supérieure de 10 000, en particulier de 5 000, de préférence de 1 000.

8. Article de prophylaxie selon l'une quelconque des revendications 1 à 7, **caractérisé en ce que** les agglomérats (9) sont d'une forme approximativement sphérique, le cas échéant la forme d'un ballon de football.

9. Article de prophylaxie de l'une quelconque des revendications 1 à 8, **caractérisé en ce que** les agglomérats (9) sont creux.

10. Article de prophylaxie selon l'une quelconque des revendications 1 à 9, **caractérisé en ce qu'**une pluralité d'agglomérats (9) se superposent.

11. Article de prophylaxie selon l'une quelconque des revendications 1 à 10, **caractérisé en ce que** les agglomérats (9) présentent une dimension/un diamètre choisis dans une plage ayant une limite inférieure de 5 µm, de préférence de 10 µm, en particulier de 15 µm, et une limite supérieure de 100 µm, de préférence de 80 µm, en particulier de 70 µm.

12. Article de prophylaxie selon l'une quelconque des revendications 1 à 11, **caractérisé en ce que** le diamètre des capsules individuelles (8) et/ou des agglomérats (9) est inférieur à 50 %, de préférence à 45 %, en particulier à 40 % de l'épaisseur de la couche de glissement.

13. Article de prophylaxie selon l'une quelconque des revendications 1 à 12, **caractérisé en ce que** les microcapsules (7) et/ou agglomérats (9) sont disposés intégrés dans la surface intérieure (3) et/ou directement adjacents à la surface intérieure (3) de la couche de glissement (2), dans la direction de la couche de support (1).

14. Article de prophylaxie selon l'une quelconque des revendications 1 à 13, **caractérisé en ce que** les capsules individuelles (8) et/ou agglomérats (9) présentent un enrobage.

15. Article de prophylaxie selon l'une quelconque des revendications 1 à 14, **caractérisé en ce que** les capsules individuelles (8) et / ou agglomérats (9) sont marqués avec un colorant.

16. Article de prophylaxie selon l'une quelconque des revendications 1 à 15, **caractérisé en ce que** les capsules individuelles (8) et/ou agglomérats (9) sont insolubles dans l'eau.

17. Article de prophylaxie selon l'une quelconque des revendications 1 à 15, **caractérisé en ce que** les capsules individuelles (8) et/ou agglomérats (9) sont solubles dans l'eau.

18. Article de prophylaxie selon l'une quelconque des revendications 1 à 17, **caractérisé en ce que** les capsules individuelles (8) et/ou agglomérats (9) sont disposés au moins par endroits dans la zone de l'avant-bras distal, du poignet, des os du carpe, des os métacarpiens et/ou des phalanges de base, médianes et terminales des doigts d'un gant médical.

19. Article de prophylaxie selon l'une quelconque des revendications 1 à 18, **caractérisé en ce que** les capsules individuelles (8) et/ou agglomérats (9) sont également disposés au moins par endroits dans la zone de la paume ainsi que dans la zone dorsale.

20. Article de prophylaxie selon l'une quelconque des revendications 1 à 19, **caractérisé en ce qu'**en utilisation, un changement dans la structure superficielle de la couche de glissement (2) survient.

21. Composition pour la formation d'une couche de glissement (2) d'un article de prophylaxie sous la forme d'un gant ou d'un préservatif, comprenant un mélange d'eau, de polyuréthane et de microcapsules (7), contenant au moins un principe actif et/ou un colorant, **caractérisé en ce qu'**au moins 90 % des microcapsules (7) ont un diamètre inférieur à 10 µm, et sont présentes à la fois en tant que capsules individuelles (8) mais aussi sous forme d'agglomérats (9).

22. Composition selon la revendication 21, **caractérisée en ce que** la concentration des microcapsules (7) est choisie dans une plage avec une limite inférieure de 0,01 %, de préférence de 0,1 %, en particulier de 0,5 %, et une limite supérieure de 10%, de préférence de 5 %, en particulier de 1 %.

23. Composition selon les revendications 21 ou 22, **caractérisée en ce qu'**elle contient de l'acrylate et/ou du butadiène carboxylé et/ou du silicone.

24. Composition selon la revendication 23, **caractérisé en ce que** le silicone présente des propriétés d'auto-réticulation.

25. Composition selon l'une quelconque des revendications 21 à 24, **caractérisé en ce que** le pH est pratiquement neutre.

26. Composition selon la revendication 25, **caractérisé en ce que** la valeur du pH est choisi dans une plage avec une limite inférieure de 5,5, de préférence de 6, en particulier de 6,5, et une limite supérieure de 8, de préférence de 7,5, en particulier de 7,2.

27. Utilisation de la composition selon l'une quelconque des revendications 21 à 26 en tant que couche de glissement (2) pour un article de prophylaxie sous la forme d'un gant, en particulier d'un gant médical, ou d'un préservatif.
